# FASCICULE DE BREVET EUROPEEN

(11) **EP 0 886 477 B1**
(45) Date de publication et mention de la délivrance du brevet: **31.07.2002**
(21) Numéro de dépôt: 97903229.9
(22) Date de dépôt: 05.02.1997
(51) Int. Cl.: A23J 3/12, A23J 1/04, A23J 1/06

(54) **AGENTS D'INHIBITION DE LA CROISSANCE DES CRISTAUX DE LA GLACE**
EISKRISTALLWACHTUMSHEMMER
ICE CRYSTAL GROWTH INHIBITING AGENTS

(30) Priorité: 09.02.1996 EP 96200309
(43) Date de publication de la demande: 30.12.1998
(73) Titulaire: SOCIETE DES PRODUITS NESTLE S.A., 1800 Vevey (CH)
(72) Inventeur: JANN, Alfred, F-74500 Publier (FR); LUNDHEIM, Rolv, N-7010 Trondheim (NO)
(74) Mandataire: Vuille, Roman
(86) Numéro de dépôt international: EP9700547
(87) Numéro de publication internationale: WO9728698

(56) Documents cités:
- WO-A-92/12722
- FOOD TECHNOLOGY, vol. 47, no. 1, 1 Janvier 1993, pages 82, 84-88, 90, XP000338468 FEENEY R E ET AL: "ANTIFREEZE PROTEINS: PROPERTIES, MECHANISM OF ACTION, AND POSSIBLE APPLICATIONS"
- POLAR BIOLOGY, vol. 1, no. 2, 1982, pages 115-123, XP000575107 SCHNEPPENHEIM, THEEDE: "FREEZING-POINT DEPRESSING PEPTIDES AND GLYCOPROTEINS FROM ARCTIC-BOREAL AND ANTARCTIC FISH"
- CANADIAN JOURNAL OF ZOOLOGY, vol. 66, no. 2, 1988, pages 2611-2617, XP000571714 DAVIES, HEW, FLETCHER: "fish antifreeze proteins: physiology and evolutionary biology"
- FOOD PROCESSING, 1992, page 55 XP000571336 SWIENTEK: "frozen foods with 'fresh' qualities"

## Description

La présente invention a pour objet des agents d'inhibition de la croissance des cristaux de la glace, un procédé de préparation de tels agents et un procédé d'utilisation de ces agents dans la fabrication d'un produit alimentaire.

Il est connu que des agents d'inhibition de la croissance des cristaux de la glace dénommés "protéines de l'hystérésis thermique" ont la capacité de se fixer sur les cristaux de la glace et de diminuer leur croissance (Biophysical Journal, February 1991, p 409 - 418). Deux propriétés de ces agents ont été mises en évidence: ils ont la capacité de diminuer la température de congélation apparente d'une solution sans en affecter sa température de décongélation et ils ont également la capacité d'inhiber la recristallisation des cristaux de la glace (Cryobiology, vol. 25, 1988, p 55 - 60).

De plus, trois types d'agents d'inhibition de la croissance des cristaux de la glace ont été mis en évidence chez certains poissons de l'Arctique et de l'Antarctique notamment (The FASEB Journal, May 1990, p 2460 - 2468). Ces agents sont de structure protéique ou de structure glycoprotéique. Ils ont été isolés du plasma ou du sérum de ces poissons puis purifiés. Mais, ils sont également présents dans d'autres tissus.

Il est également connu d'utiliser des agents d'inhibition de la croissance des cristaux de la glace pour améliorer la qualité de produits alimentaires, tels que les desserts congelés, les pâtes congelées ou les produits frais, comme les tomates. DNA Plant Technology Corp a synthétisé une protéine artificielle qui est un agent d'inhibition de la croissance des cristaux de la glace, semblable à ceux que l'on peut trouver chez certains poissons ou d'autres organismes pouvant vivre dans des conditions de températures très basses (Food Processing, October 1992, p 55).

Mais, à ce jour, aucun agent d'inhibition de la croissance des cristaux de la glace n'a été isolé chez *Zoarces viviparus,* poisson vivant notamment sur les côtes de la Norvège et sur les côtes de la mer baltique.

La présente invention a pour but de proposer de nouveaux agents d'inhibition de la croissance des cristaux de la glace, présentant un niveau d'activité remarquable et peuvant être avantageusement utilisés notamment dans la congélation ou la texturation de produits, tels que les produits alimentaires, par exemple.

A cet effet, les agents d'inhibition de la croissance des cristaux de la glace selon la présente invention sont des agents extraits de *Zoarces viviparus* ayant un poids moléculaire de 4-5,5 kDa susceptibles d'être obtenus par un procédé, dans lequel on prélève du sérum de *Zoarces viviparus*, on isole les protéines du sérum, on sépare les protéines par filtration sur gel et l'on isole la fraction protéique ayant une hystérésis thermique de 1-1,8° C.
De préférence, ces agents d'inhibition de la croissance des cristaux de la glace présentent une hystérésis thermique de 1,3-1,5° C.

On a constaté avec surprise que ces agents d'inhibition de la croissance des cristaux de la glace permettent effectivement de diminuer la taille des cristaux de glace, lors de la congélation de produits alimentaires, tels que les glaces, les desserts congelés ou les pâtes congelées, par exemple, et confèrent ainsi à ces derniers une texture plus onctueuse.

Dans la suite de la description, on emploiera l'expression "agent d'inhibition des cristaux" dans le sens d"'agent d'inhibition de la croissance des cristaux de la glace".

Dans la suite de la description, on emploiera l'expression "taille typique des cristaux" dans le sens de "taille des cristaux de la glace retrouvée majoritairement".

Dans la suite de la description, on emploiera l'expression "diamètre équivalent" dans le sens de "diamètre d'un cercle qui a la même surface que l'image du cristal considéré".

Dans le procédé de préparation d'un extrait d'agents d'inhibition de la croissance des cristaux de la glace selon la présente invention, on prépare du sérum de *Zoarces viviparus* contenant lesdits agents.

Pour ce faire, on peut extraire le sang de *Zoarces viviparus*, on peut le refroidir, on peut recueillir le surnageant constituant le sérum de *Zoarces viviparus* contenant les agents d'inhibition de la croissance des cristaux de la glace, puis on peut congeler le surnageant, par exemple.

On peut extraire le sang de *Zoarces viviparus* à l'aide d'un capillaire traité, notamment un capillaire traité à l'héparine de sodium, par exemple.

On peut refroidir le sang de *Zoarces viviparus à* 0-5° C, de manière, notamment, à inhiber l'activité des protéases et des peptidases contenues dans le sang, par exemple.

On peut recueillir le surnageant constituant le sérum de *Zoarces viviparus* contenant les agents d'inhibition de la croissance des cristaux de glace, en centrifugeant le sang de *Zoarces viviparus* à 1000-5000 g à 0-5° C pendant 5-15 min, par exemple. Pour ce faire, on peut utiliser une centrifugeuse du type Heraeus Minifuge GL, commercialisée par Heraeus SA, 23, route des Jeunes, CH - 1227 Carouge-Genève par exemple.

On peut congeler le surnageant à une température comprise entre -15° C et - 40° C, par exemple.

La présente invention concerne également un procédé pour diminuer la taille des cristaux de la glace, dans lequel on ajoute à un produit alimentaire au moins agent d'inhibition de la croissance des cristaux de la glace extrait de *Zoarces viviparus.* On peut notamment ajouter 0,01-10% d'agents d'inhibition de la croissance des cristaux de la glace selon la présente invention à un produit alimentaire au cours de sa préparation.

On peut ensuite congeler le produit alimentaire ainsi préparé, par exemple. On peut notamment le congeler à une température comprise entre - 15° C et - 40° C.

Les agents d'inhibition de la croissance des cristaux selon la présente invention sont décrits plus en détails à l'aide de données biochimiques et par l'intermédiaire des différentes propriétés déterminées notamment à l'aide de différents tests ci-après. Les pourcentages sont donnés en poids, sauf indication contraire.

### Mise en évidence de la cristallisation des cristaux de la glace dans une solution de saccharose à 20% en présence de sérum de Zoarces viviparus

On prépare 6 échantillons de 1 ml d'une solution de saccharose à 20%, auxquels on ajoute, à différentes concentrations, du sérum de *Zoarces viviparus,* contenant les agents d'inhibition des cristaux.

Les échantillons sont gardés au réfrigérateur, avant d'être placés sous un microscope de type Polyvar, commercialisé par Reichert-Jung, Harnalser Hauptstrasse 219, AT - 1170 Wien et d'être refroidis rapidement jusqu'à une température de - 100° C à l'aide d'un contrôleur de températures de type Lincam, commercialisé par Lincam Scientific Instruments LTD, Epsom Downs Metro Centre, Waterfield, Tadworth, Surrey, KT205HT - UK.

Puis l'on réchauffe les échantillons jusqu'à une température de -9° C et l'on observe, au microscope de type Polyvar, la taille des cristaux contenus dans les échantillons ainsi préparés dans un intervalle de temps de 30 à 120 min.

Parallèlement, on prépare, dans les mêmes conditions, un échantillon témoin contenant 1 ml d'une solution de saccharose à 20%.

Les % de sérum de *Zoarces viviparus* contenant les agents d'inhibition des cristaux que l'on ajoute aux échantillons d'une solution de saccharose à 20%, sont donnés en volume.

Echantillon 1: A 1 ml d'une solution de saccharose à 20%, on ajoute 1,3% de sérum de *Zoarces viviparus* puis l'on prépare l'échantillon, comme indiqué ci-dessus. Après 60 min à -9° C, les cristaux ont une taille typique inférieure à 2 µm. On n'observe aucune évolution de la taille des cristaux au cours du temps.

Echantillon 2: A 1 ml d'une solution de saccharose à 20%, on ajoute 1% de sérum de *Zoarces viviparus* puis l'on prépare l'échantillon, comme indiqué ci-dessus. Après 30 min à -9° C, les cristaux ont une taille typique inférieure à 2 µm. On n'observe aucune évolution de la taille des cristaux au cours du temps.

Echantillon 3: A 1 ml d'une solution de saccharose à 20%, on ajoute 0,1% de sérum de *Zoarces viviparus* puis l'on prépare l'échantillon, comme indiqué ci-dessus. Après 30 min à -9° C, les cristaux ont une taille typique inférieure à 15 µm. La taille des cristaux évolue très peu au cours du temps. Après 120 min à -9° C, la taille typique des cristaux reste inférieure à 15 µm et les cristaux présentent des formes avec des angles prononcés.

Echantillon 4: A 1 ml d'une solution de saccharose à 20%, on ajoute 0,02% de sérum de *Zoarces viviparus* puis l'on prépare l'échantillon, comme indiqué ci-dessus. La taille des cristaux évolue légèrement entre 60 min et 90 min à -9°C. La taille typique des cristaux est inférieure à 20 µm après 90 min à -9° C. Les cristaux présentent des formes avec des angles prononcés.

Echantillon 5: A 1 ml d'une solution de saccharose à 20%, on ajoute 0,013% de sérum de *Zoarces viviparus* puis l'on prépare l'échantillon, comme indiqué ci-dessus. On observe la même évolution de la taille des cristaux que pour l'échantillon préparé avec 0,02% de sérum de *Zoarces viviparus.* La taille typique des cristaux est supérieure à 20 µm après 90 min à -9° C. Les cristaux présentent des formes avec des angles légèrement arrondis.

Echantillon 6: A 1 ml d'une solution de saccharose à 20%, on ajoute 0,01% de sérum de *Zoarces viviparus* puis l'on prépare l'échantillon, comme indiqué ci-dessus. On observe une évolution de la taille des cristaux plus importante que dans l'échantillon préparé avec 0,013% de serum de *Zoarces viviparus*. De plus, les cristaux ont des formes plus arrondies que dans l'échantillon préparé avec 0,013% de sérum de *Zoarces viviparus.* La taille typique des cristaux est supérieure à 25 µm, après 90 min à -9° C

Echantillon témoin: On prépare une solution de saccharose à 20% comme indiqué ci-dessus mais sans y ajouter du sérum de *Zoarces viviparus.* Les cristaux grandissent vite. Leur forme est arrondie et après 90 min à -9° C, la taille typique des cristaux est supérieure à 25 µm.

Ainsi, si l'on ajoute à une solution de saccharose à 20% du sérum de *Zoarces viviparus* à une concentration de 1,3 à 0,013%, on observe au microscope des cristaux de glace relativement petits et dont la taille n'évolue que très peu au cours du temps.

Par contre, si l'on ajoute à une solution de saccharose à 20% une très faible concentration de sérum de *Zoarces viviparus,* les cristaux de glace grandissent plus rapidement.

Entre l'échantillon 6 et l'échantillon témoin, on est à la limite où l'on observe très peu de différences dans la forme et dans l'évolution de la taille des cristaux de glace.

Si la concentration de sérum de Zoarces viviparus contenu dans une solution de saccharose à 20% est comprise entre 0,01% et 10%, on constate que les formes des cristaux présentent des angles plus prononcés que celles des cristaux d'une solution témoin ne contenant pas ledit sérum.

### Mesure de l'hystérésis thermique

Cette analyse est effectuée à l'aide d'un contrôleur de températures de type Clifton Nanolitre Osmomètre, commercialisé par Clifton Technical Physics, P.O. Box, Hartford, US - New York, 12830 et d'un stéréomicroscope de type Wild MZ8, commercialisé par Leica A.G, Verkaufsgesellschaft, Kanalstrasse 21, CH - 8152 Glattbrugg.

Pour ce faire, on prélève une fraction de 20 ml d'un échantillon de sérum de *Zoarces viviparus* à température ambiante et on la dépose dans un réceptacle placé dans le contrôleur de températures. Ledit réceptacle contient de la paraffine liquide. On effectue une congélation rapide suivie d'une décongélation progressive, on observe à l'aide d'un stéréomicroscope la décongélation des cristaux. Puis l'on ralenti et l'on arrête la décongélation, de manière à stabiliser le seul cristal de petite taille restant et l'on note alors la température. Cette température est le point de décongélation. Puis, l'on refroidit doucement et l'on note la température à laquelle le cristal commence à grossir. Cette température est le point de congélation apparente.

On calcule alors la valeur de l'hystérésis thermique en calculant la différence entre le point de congélation apparente et le point de décongélation. La valeur de l'hystérésis thermique de la fraction de sérum de *Zoarces viviparus* est de 1,4° C, ce qui correspond à une valeur élevée pour un poisson contenant de tels agents.

### Caractérisation des agents d'inhibition de la croissance des cristaux de la glace extraits de Zoarces viviparus

On caractérise les agents d'inhibition de la croissance des cristaux de la glace extraits de *Zoarces viviparus* par analyse chromatographique suivie d'une séparation par électrophorèse sur gel de polyachrylamide.

On effectue donc tout d'abord une analyse chromatographique. Pour ce faire, on sépare les protéines de 20 mg contenues dans le sérum de *Zoarces viviparus* sur une colonne superose 12, commercialisée par Pharmacia Biotech A.G, Dübendorf, CH, avec une pompe Waters 519 et un détecteur DAD Waters 990, commercialisés par Waters A.G, Volketswil, CH. Pour effectuer la séparation par chromatographie liquide, on utilise comme phase mobile, un tampon de carbonnate d'ammonium, 150 mM. pH 7,8 à un flux de 0,5 ml/min.

A partir des fractions éluées par chromatographie, on effectue une mesure de l'hystérésis thermique telle que décrite dans le test "mesure de l'hystérésie thermique". On met ainsi en évidence le fait que la fraction éluant entre 30 et 33 minutes est celle qui contient les agents d'inihibition de la croissance des cristaux de la glace de *Zoarces viviparus,* puis que c'est dans cette fraction que l'on mesure le maximum de l'hystérésis thermique.

On concentre et l'on sépare alors par électrophorèse sur gel cette fraction active en hystérésie thermique. On effectue cette séparation à 100V pendant 100 min sur un gel préfabriqué, Ready gels, commercialisé par BIO-RAD, Glattbrugg, CH, avec un appareil Mini Protean II, commercialisé par BIO-RAD, Glattbrugg, CH.

Après coloration, on évalue le poids moléculaire des protéines contenues dans cette fraction, par comparaison avec une gamme de standards de poids moléculaire compris entre 26,6 et 1,4 kDa, commercialisée par BIO-RAD, Glattbrugg, CH, séparés sur le même gel.

On met ainsi en évidence le fait que les protéines de la fraction active en hystérésis thermique ont un poids moléculaire de 4-5,5 kDa.

### Etude de l'influence de la chaleur sur l'activité des agents d'inhibition de la formation des cristaux contenus dans le sérum de Zoarces viviparus

On chauffe des échantillons de sérum de *Zoarces vivivparus* pendant 2 h à 40° C, à 50° C, à 60° C, à 70° C et à 80° C puis l'on mesure l'hystérésis thermique de ces échantillons. On compare les valeurs de l'hystérésis thermique de ces différents échantillons avec la valeur de l'hystérésis thermique d'un échantillon de sérum de *Zoarces viviparus* gardé à température ambiante.

Pour ce faire, on procède de la manière décrite dans le test "Mesure de l'hystérésis thermique".

Les valeurs de l'hystérésis thermique de ces échantillons de sérum sont mentionnées dans le tableau I ci-après.

**Tableau I**

| **Echantillon traité à la chaleur** | **valeur de l'hystérésis thermique (° C)** |
|---|---|
| échantillon témoin traité 2h à 20° C | 0,232 |
| échantillon traité 2h à 40° C | 0,208 |
| échantillon traité 2h à 50° C | 0,186 |
| échantillon traité 2h à 60° C | 0,204 |
| échantillon traité 2h à 70° C | 0,192 |
| échantillon traité 2h à 80° C | 0,204 |

Les valeurs de l'hystérésis thermique ainsi mesurées mettent en évidence le fait que les agents d'inhibition des cristaux contenus dans le sérum de *Zoarces viviparus* présentent une très grande stabilité à la chaleur. Ceci est un avantage pour l'utilisation de tels agents dans des procédés de fabrication alimentaire faisant intervenir une étape à température élevée, notamment dans des procédés faisant intervenir une étape de pasteurisation.

### Mise en évidence de l'influence de la concentration de sérum de Zoarces viviparus sur la valeur de l'hystérésie thermique

On dilue des échantillons de sérum de *Zoarces viviparus* dans de l'eau bi-distillée et l'on mesure la valeur de l'hystérésie thermique de ces échantillons, de la manière décrite dans le test "Mesure de l'hystérésis thermique".

Les valeurs de l'hystérésis thermique de ces échantillons de sérum sont mentionnées dans le tableau II ci-après.

**Tableau II**

| **Echantillon dilué dans de l'eau bi-distilée** | **valeur de l'hystérésis thermique (°C)** |
|---|---|
| échantillon témoin | 0,524 |
| échantillon dilué au 1/2 | 0,276 |
| échantillon dilué au 1/4 | 0,164 |
| échantillon dilué au 1/8 | 0,094 |

Les résultats ainsi obtenus mettent en évidence le fait que la valeur de l'hystérésis thermique est dépendente de la concentration de l'échantillon de sérum de *Zoarces viviparus.* Cette dépendence n'est pas linéaire.

### Etude comparative de l'activité d'agents d'inhibition de la formation des cristaux contenus dans un sérum de poisson

On mesure l'hystérésis thermique d'échantillons de sérum extrait de *Gadus morhua*, poisson vivant sur les côtes de Terre-Neuve, *Pollachius pollachius,* poisson vivant sur les côtes de la Méditerranée jusqu'au nord de la Norvège, *Gobiuscullus flavescens,* poisson vivant sur les côtes européennes et sur les côtes japonnaises, *Myoxocephalus scorpius*, poisson vivant dans l'Arctique et près des côtes de la Norvège, *Liparis liparis*, poisson vivant sur les côtes de l'Atlantique et *Spinachia spinachia,* poisson vivant dans la baie de Biscay et jusqu'au nord de la Norvège. On compare les valeurs de l'hystérésis thermique de ces différents échantillons avec la valeur de l'hystérésis thermique d'un échantillon de sérum de *Zoarces viviparus.*

Tous les poissons ont été capturés en hivers dans un fjord norvégien et ont été tués au maximum 3 h avant d'effectuer les mesures de l'hystérésis thermique à partir d'échantillons de sérum extraits de ces poissons.

Pour ce faire, on procède de la manière décrite dans le test "Mesure de l'hystérésis thermique".

Les valeurs de l'hystérésis thermique de ces échantillons de sérum sont mentionnées dans le tableau III ci-après.

**Tableau III**

| **échantillon de sérum extrait de** | **valeur de l'hystérésis thermique (° C)** |
|---|---|
| *Gadus morhua* | 0 |
| *Pollachius pollachius* | 0 |
| *Gobiuscullus flavescens* | 0,4 |
| *Myoxocephalus scorpius* | 0,1 |
| *Spinachia spinachia* | 0,2 |
| *Liparis liparis* | 0,4 |
| *Zoarces viviparus* | 1,4 |

Les valeurs de l'hystérésis thermique ainsi mesurées mettent en évidence le fait que les agents d'inhibition des cristaux contenus dans le sérum de *Zoarces viviparus* présentent une activité supérieure à celles des agents d'inhibition des cristaux contenus dans le sérum d'autres poissons vivant dans des conditions similaires.

### Mesure de la taille des cristaux de la glace dans la crème glacée contenant du sérum de Zoarces viviparus

On mesure la taille des cristaux de la glace dans une crème glacée contenant du sérum de *Zoarces viviparus,* puis l'on compare ces résultats avec la taille de cristaux de la glace dans une crème glacée produite dans des conditions identiques, mais ne contenant pas de sérum de *Zoarces viviparus.*

Pour ce faire, on utilise une crème glacée contenant 0,05% de sérum de *Zoarces viviparus* que l'on a stocké à -40° C. On scie des portions de 1cm³ au milieu de la masse de la crème glacée que l'on transfère dans une enceinte réfrigérée à -10° C.

Dans un tube de caoutchouc silicone on prépare, à -10° C, un échantillon contenant 3 mm³ de graisse silicone, 3 mm³ d'une desdites portions et 7 gouttes de benzine de pétrole. On ferme le tube aux deux extrémités et l'on mélange son contenu à l'aide d'une tige en verre.

On transfère l'échantillon sur une lame porte-objet de microscope et on le recouvre d'une seconde lame porte-objet de microscope.

On place l'échantillon ainsi préparé sous le microscope optique et l'on visualise son image sur écran vidéo. A l'aide du programme *PC-IMAGE* commercialisé par Gloor Instrumente AG, Brauereistrasse 10, CH - 8610 Uster, on obtient une image binaire représentative des cristaux. A l'aide dudit programme, on mesure la taille des cristaux exprimée en diamètre équivalent.

On mesure la taille des cristaux de la glace dans un témoin que l'on prépare comme décrit ci-dessus à partir d'une crème glacée ne contenant pas de sérum de *Zoarces viviparus.*

Puis l'on compare, selon le critère de la taille, la distribution des cristaux de la glace dans la crème glacée contenant du sérum de *Zoarces viviparus* par rapport au témoin.

La distribution des cristaux de la glace dans la crème glacée contenant du sérum de *Zoarces viviparus (a)* et celle des cristaux de la glace dans le témoin (b), selon le critère de la taille, est indiquée dans le tableau IV ci-après.

**Tableau IV**

| taille des cristaux (µm) | distribution dans (a) | distribution dans (b) |
|---|---|---|
| 0-10 | 1 | 0 |
| 10-20 | 162 | 50 |
| 20-30 | 192 | 132 |
| 30-40 | 116 | 128 |
| 40-50 | 50 | 76 |
| 50-60 | 14 | 27 |
| 60-70 | 12 | 12 |
| 70-80 | 0 | 4 |
| 80-90 | 3 | 0 |
| 90-100 | 0 | 0 |
| 100-110 | 0 | 0 |

On constate, à partir des résultats mentionnés dans le tableau IV, que les cristaux de petite taille sont présents en plus grand nombre dans la crème glacée contenant du sérum de *Zoarces viviparus* (a). En effet, on peut mettre en évidence le fait que, dans la crème glacée contenant du sérum de *Zoarces viviparus* (a), les cristaux sont distribués majoritairement dans une fourchette de tailles allant de 10 à 40 µm, alors que, dans le témoin (b), les cristaux sont distribués majoritairement dans une fourchette de tailles allant de 20 à 50 µm.

De plus, à partir des valeurs de taille de l'ensemble des cristaux, on calcule la taille moyenne des cristaux dans la crème glacée contenant du sérum de *Zoarces viviparus* et l'on compare cette valeur à la taille moyenne des cristaux de la glace dans le témoin. La taille moyenne des cristaux de la glace dans la crème glacée contenant du sérum de *Zoarces viviparus* est de 27,8 µm et celle des cristaux de la glace dans le témoin est de 33,8 µm.

### Mesure de la taille des cristaux de la glace dans la crème glacée contenant du sérum de Zoarces viviparus après un choc thermique

On soumet la crème glacée contenant du sérum de *Zoarces viviparus* et le témoin à un choc thermique pendant 36 h. Le choc thermique consiste en un cycle de températures, au cours duquel on réchauffe à -4° C puis l'on refroidit à -20° C et enfin on réchauffe et l'on refroidit dans l'intervalle de températures compris entre -4° C et -20° C.

On procède alors de la manière décrite dans le test ci-dessus.

On compare, selon le critère de la taille, la distribution des cristaux de la glace dans la crème glacée contenant du sérum de *Zoarces viviparus* par rapport à celle des cristaux de la glace dans le témoin.

La distribution des cristaux de la glace dans la crème glacée contenant du sérum de *Zoarces viviparus (c)* et celle des cristaux de la glace dans le témoin (d), selon le critère de la taille, est indiquée dans le tableau V ci-après.

**Tableau V**

| taille des cristaux (µm) | distribution dans (c) | distribution dans (d) |
|---|---|---|
| 0-10 | 0 | 0 |
| 10-20 | 1 | 1 |
| 20-30 | 37 | 0 |
| 30-40 | 61 | 2 |
| 40-50 | 61 | 8 |
| 50-60 | 40 | 20 |
| 60-70 | 18 | 25 |
| 70-80 | 14 | 40 |
| 80-90 | 10 | 37 |
| 90-100 | 3 | 41 |
| 100-110 | 6 | 30 |
| 110-120 | 6 | 18 |
| 120-130 | 4 | 18 |
| 130-140 | 1 | 9 |
| 140-150 | 0 | 8 |
| 150-160 | 0 | 6 |
| 160-170 | 0 | 8 |
| 170-180 | 0 | 1 |
| 180-190 | 0 | 3 |
| 190-200 | 0 | 3 |

Les résultats mentionnés dans le tableau V mettent en évidence le fait que, après un choc thermique, les cristaux de la glace dans une crème glacée à laquelle on ajoute du sérum de *Zoarces viviparus* contenant les agents d'inhibition de la croissance des cristaux sont de taille inférieure à celle des cristaux de la glace de la crème glacée à laquelle on n'a pas ajouté ledit sérum.

En effet, en présence de sérum de *Zoarces viviparus,* 76% des cristaux de la glace dans une crème glacée sont de taille inférieur à 60 µm. Alors que dans le témoin, seulement 11% des cristaux de la glace sont de taille inférieure à 60 µm.

De plus, en présence de sérum de *Zoarces viviparus,* seulement 6% des cristaux de la glace dans une crème glacée sont de taille supérieure à 100 µm. Alors que dans le témoin, 38% des cristaux sont de taille supérieure à 100 µm.

Enfin, à partir des valeurs de taille de l'ensemble des cristaux on calcule la taille moyenne des cristaux de la glace dans la crème glacée contenant du sérum de *Zoarces viviparus* et ayant subi un choc thermique. Puis, l'on compare cette valeur à la taille moyenne des cristaux de la glace dans le témoin. La taille moyenne des cristaux de la glace dans la crème glacée contenant du sérum de *Zoarces viviparus* est de 50,8 µm et celle des cristaux de la glace dans le témoin est de 96,5 µm.

On met donc en évidence le fait que la recristallisation des cristaux de la glace est partiellement inhibée dans une crème glacée à laquelle on a ajouté du sérum de *Zoarces viviparus* contenant les agents d'inhibition de la croissance des cristaux et à laquelle on a fait subir ensuite un choc thermique.

### Analyse de la texture d'un produit alimentaire auquel on a ajouté un extrait de sérum de Zoarces viviparus

Pour analyser la texture d'un produit alimentaire, on mesure, à l'aide d'un analyseur de texture, notamment l'Instron Food Testing Instrument commercialisé par Instron Limited, Coronation Road, High Wycombe, Bucks HP12 3SY, UK, l'énergie necessaire pour le casser.

Pour ce faire, on place le produit alimentaire sur un plateau circulaire évidé en son milieu d'un disque de 40 mm de diamètre et l'on presse un piston présentant une base circulaire de 30 mm de diamètre, sur la face supérieur du produit alimentaire, tout en mesurant l'énergie pour le casser.

On mesure l'énergie nécessaire pour casser des filets de cabillaud émincés auxquels on a ajouté 1 g d'extrait de sérum de *Zoarces viviparus* contenant des agents d'inhibition de la croissance des cristaux puis congelés et cuits. On compare alors ces mesures à celles effectuées sur des filets de cabillaud émincés sans addition d'extrait de sérum de *Zoarces viviparus* que l'on a également congelés puis cuits.

### Exemple comparatif

On mélange donc 1 g d'extrait de sérum de *Zoarces viviparus* dans 25 g d'eau et l'on ajoute cette solution à 1 kg de filets de cabillaud émincés. On mélange bien le tout que l'on divise ensuite en portions de 40 g. Puis, on congèle ces portions à - 35° C.

Par comparaison, on mélange 25 g d'eau à 1 kg de filets de cabillaud émincés que l'on divise ensuite en portions de 40 g, de manière à réaliser des témoins. Ces témoins sont également congelés à - 35° C.

On dispose ces portions de 40 g et les témoins dans des sachets en plastique puis on les chauffe à une température de 70° C.

Puis, on mesure l'énergie nécessaire pour casser les portions et les témoins. On compare alors les valeurs mesurées.

L'énergie nécessaire pour casser des filets de cabillaud émincés auxquels on a ajouté de l'extrait de sérum de *Zoarces viviparus* est de 10,9 +/- 0,3 J/100 g alors que celle nécessaire pour casser des filets de cabillaud sans addition d'extrait de sérum de *Zoarces viviparus* est de 12 +/- 0,3 J/100 g.

Ces résultats mettent en évidence le fait que les filets de cabillauds émincés, auxquels on a ajouté de l'extraitdu sérum de *Zoarces viviparus* et que l'on a congelés, présentent une texture plus tendre après cuisson que les filets de cabillaud émincés puis congelés sans addition d'extrait de sérum de *Zoarces viviparus.*

### Analyse microscopique de la texture d'un produit alimentaire solide

Pour effectuer une analyse microscopique de la texture, on utilise un microscope réfrigéré afin d'évaluer la taille des cristaux de glace présents.

Pour ce faire, on prépare des portions de 40 g de filets de cabillaud et des témoins, de la manière décrite dans l'exemple comparatif ci-dessus, que l'on congèle dans un congélateur à air pulsé à une température de - 35° C.

Puis, on observe des échantillons de ces portions de 40 g congelées et des échantillons des témoins congelés sous un microscope réfrigéré, de manière à évaluer et à comparer la taille des cristaux de glaces de ces différents échantillons.

On met en évidence le fait que la taille des cristaux de glace contenus dans les filets de cabillaud émincés, auxquels on a ajouté de l'extrait de sérum de *Zoarces viviparus* contenant des agents d'inhibition de la croissance des cristaux de la glace et que l'on a ensuite congelé, est inférieure à celle des cristaux de glace contenus dans les filets de cabillaud émincés et congelés sans addition d'extrait de sérum de *Zoarces viviparus.*

L'addition d'extrait de sérum de *Zoarces viviparus* permet donc d'éviter les dommages causés par l'augmentation de la taille des cristaux de glace au cours de la congélation notamment d'un produit alimentaire solide ou fibreux.

L'exemple suivant est présenté à titre d'illustration d'une utilisation industrielle, dans le domaine alimentaire, des agents d'inhibition de la croissance des cristaux selon la présente invention. Dans l'exemple ci-après, les pourcentages sont donnés en poids, sauf indication contraire.

### Exemple 1

On utilise du sérum de *Zoarces viviparus* contenant les agents d'inhibition de la croissance des cristaux pour la fabrication d'une crème glacée.

Pour ce faire, on dissout dans 494 g d'eau à 65° C, 92,3 g de lait écrémé en poudre, 150 g de saccharose, 26,2 g de sirop de glucose et 5 g d'émulsifiant.

On y ajoute 4 g d'arôme de vanille et 228,5 g de crème à 35% de matière grasse.

On homogénéise cette préparation dans un homogénéisateur de type Rannie, commercialisé par Kindler Maschinen AG, Postfach 297, CH-8021 Zürich, en deux passages successifs, le premier à 140 bar et le second à 40 bar.

On pasteurise la préparation homogénéisée à 83° C pendant 30 s dans un échangeur à plaques.

On la refroidit à 4° C et on la laisse reposer 12 h à cette température, avant d'ajouter 0,05% de sérum de *Zoarces viviparus* et d'effectuer le glaçage dans un freezer de type HOYER MF50 commercialisé par APV TECHNOHOY, Axel Kiers Vej 28-30, DK-8270 Aarhus-Hojbjerg

On obtient ainsi une crème glacée présentant une texture mousseuse.

On durcit ensuite cette crème glacée dans une cellule de refroidissement à air pulsé et on la stocke à -35° C.

Après tempérage à -18° C, cette crème glacée présente une texture lisse et onctueuse.

## Revendications

1. Agents d'inihibition de la croissance des cristaux de la glace ayant un poids moléculaire de 4-5,5 kDa susceptibles d'être obtenus par un procédé, dans lequel on prélève du sérum de *Zoarces viviparus,* on isole les protéines du sérum, on sépare les protéines par filtration sur gel et l'on isole la fraction protéique ayant une hystérésis thermique de 1-1,8° C.

2. Agents d'inhibition de la croissance des cristaux de la glace selon la revendication 1, présentant une hystérésis thermique de 1,3-1,5° C.

3. Procédé de préparation d'un extrait d'agents d'inhibition de la croissance des cristaux de la glace, dans lequel on prépare du sérum de *Zoarces viviparus* contenant lesdits agents.

4. Procédé selon la revendication 3, dans lequel:
- on extrait le sang de *Zoarces vivparus,*
- on le refroidit,
- on recueille le surnageant constituant le sérum de *Zoarces viviparus* contenant les agents d'inhibition de la croissance des cristaux de la glace,
- puis l'on congèle le surnageant.

5. Procédé pour diminuer la taille des cristaux de la glace dans lequel on ajoute à un produit alimentaire au moins un agent d'inhibition de la croissance des cristaux de la glace extrait de *Zoarces viviparus* selon l'une des revendications 1 et 2 ou obtenu par le procédé selon les revendication 3-4.

6. Procédé selon la revendication 5, dans lequel on ajoute 0,01-10% d'agents d'inhibition de la croissance des cristaux de la glace.

7. Procédé selon la revendication 5, dans lequel on congèle le produit alimentaire auquel on a ajouté au moins un agent d'inhibition de la croissance des cristaux de la glace.

8. Procédé selon la revendication 7, on congèle le produit alimentaire à une température comprise entre -15° C et - 40° C.

## Patentansprüche

1. Mittel zur Hemmung des Wachstums von Eiskristallen mit einem molekularen Gewicht von 4-5,5 kDa, die nach einem Verfahren erhältlich sind, bei dem man Serum von *Zoarces viviparus* entnimmt, die Proteine des Serums isoliert, die Proteine durch Gelfiltration abtrennt und die Proteinfraktion mit einer thermischen Hysterese von 1-1,8°C isoliert.

2. Mittel zur Hemmung des Wachstums von Eiskristallen nach Anspruch 1 mit einer thermischen Hysterese von 1,3-1,5°C.

3. Verfahren zur Herstellung eines Extrakts von Mitteln zur Hemmung des Wachstums von Eiskristallen, bei dem man Serum von *Zoarces viviparus* herstellt, das diese Mittel enthält.

4. Verfahren nach Anspruch 3, bei dem man
- das Blut von *Zoarces viviparus* extrahiert,
- es kühlt,
- den Überstand gewinnt, der das *Zoarces viviparus*-Serum bildet, das die Mittel zur Hemmung des Wachstums von Eiskristallen enthält,
- dann den Überstand tiefgefriert.

5. Verfahren zur Verringerung der Größe von Eiskristallen, bei dem man einem Nahrungsmittelprodukt mindestens ein aus *Zoarces viviparus* extrahiertes Mittel zur Hemmung des Wachstums von Eiskristallen nach einem der Ansprüche 1 und 2, oder das nach dem Verfahren nach den Ansprüchen 3 bis 4 hergestellt wurde, zusetzt.

6. Verfahren nach Anspruch 5, bei dem man 0,01-10% Mittel zur Hemmung des Wachstums von Eiskristallen zusetzt.

7. Verfahren nach Anspruch 5, bei dem man das Nahrungsmittelprodukt, dem man mindestens ein Mittel zur Hemmung des Wachstums von Eiskristallen zugesetzt hat, tiefgefriert.

8. Verfahren nach Anspruch 5, bei dem man das Nahrungsmittelprodukt auf eine Temperatur zwischen -15°C und -40°C tiefgefriert.

## Claims

1. Ice crystal growth inhibiting agents having a molecular weight of 4-5.5 kDa capable of being obtained by a method wherein the serum of *Zoarces viviparus* is extracted, the serum proteins are isolated, the proteins are separated by filtration on a gel and the protein fraction is isolated having a thermal hysteresis of 1-1.8°C.

2. Ice crystal growth inhibiting agents according to claim 1, having a thermal hysteresis of 1.3-1.5°C.

3. Method for preparing an extract of ice crystal growth inhibiting agents, wherein the serum of *Zoarces viviparus* is prepared containing the said agents.

4. Method according to claim 3, wherein:
- the blood of *Zoarces viviparus* is extracted,
- it is cooled,
- the supernatant is collected consisting of the serum of *Zoarces viviparus* containing the ice crystal growth inhibiting agents,
- the supernatant is then frozen.

5. Method for reducing the size of ice crystals wherein there is added to a food product at least one ice crystal growth inhibiting agent extracted from *Zoarces viviparus* according to either of claims 1 or 2 or obtained by the method according to claims 3-4.

6. Method according to claim 5, wherein 0.01-10 % of ice crystal growth inhibiting agents are added.

7. Method according to claim 5, wherein the food product is frozen to which at least one ice crystal growth inhibiting agent has been added.

8. Method according to claim 7, the food product being frozen at a temperature of between -15°C and -40°C.
